# EUROPÄISCHE PATENTSCHRIFT

(11) **EP 3 046 308 B1**
(45) Veröffentlichungstag und Bekanntmachung des Hinweises auf die Patenterteilung: **02.10.2019**
(21) Anmeldenummer: 15151598.8
(22) Anmeldetag: 19.01.2015
(51) Int. Cl.: H04L 29/08, H04L 12/24, G16H 40/63, G06Q 10/10, G06Q 50/22, G06F 16/93

(54) **VERFAHREN ZUM KONFIGURIEREN EINES ZAHNÄRZTLICHEN PRAXISNETZWERKS**
METHOD FOR CONFIGURING A DENTAL PRACTICE NETWORK
PROCÉDÉ DE CONFIGURATION D'UN RÉSEAU DE CABINETS DENTAIRES

(43) Veröffentlichungstag der Anmeldung: 20.07.2016
(73) Patentinhaber: Kaltenbach & Voigt GmbH, 88400 Biberach (DE)
(72) Erfinder: Hoffer, Heiko, 89601 Justingen (DE); Ulmer, Torsten, 88447 Birkenhard (DE); Speckhardt, Alto, 89264 Weißenhorn (DE)
(74) Vertreter: Thun, Clemens

(56) Entgegenhaltungen:
- US-A1- 2008 140 815
- US-A1- 2009 125 816
- US-A1- 2009 300 226
- US-A1- 2011 145 373
- US-A1- 2012 117 205

## Beschreibung

Die vorliegende Erfindung betrifft ein Verfahren zum Konfigurieren des Netzwerks einer zahnärztlichen Praxis. Insbesondere betrifft die Erfindung ein Verfahren, mit dessen Hilfe netzwerkfähige Komponenten bzw. netzwerkfähiges Equipment der Dentalpraxis in einfacher Weise identifiziert und verknüpft werden kann, ohne dass hierfür detaillierte Kenntnisse der Netzwerktopologie oder IT-Kenntnisse erforderlich wären.

Die Nutzungsmöglichkeiten verschiedener Geräte einer zahnärztlichen Praxis haben sich in den letzten Jahren dramatisch verändert. Zurückzuführen ist dies darauf, dass eine Vielzahl der genutzten Geräte zwischenzeitlich in der Lage ist, Daten auszutauschen bzw. an andere Komponenten der Praxis zu übermitteln, wodurch das Verwalten patientenspezifischer Informationen aber auch das Veranschaulichen von Untersuchungs- oder Behandlungsergebnissen deutlich verbessert wird. Ein Beispiel hierfür stellt die Verknüpfung eines Dental-Behandlungs- oder Untersuchungsplatzes mit einem separaten Rechner, einem sog. Hinterkopf-PC dar, auf dem bspw. eine Patientenverwaltungssoftware installiert ist. Beide Einheiten könnten grundsätzlich unabhängig voneinander betrieben werden, eine Kommunikation zwischen beiden Komponenten ermöglicht allerdings bspw. einerseits auf Anzeigemitteln des Behandlungs- oder Untersuchungsplatzes Patienteninformationen (z.B. Namen, Alter, zu berücksichtigende Krankheiten usw.) darzustellen sowie andererseits durchgeführte Behandlungen und/oder Untersuchungen automatisch an dem Hinterkopf-PC zu protokollieren und Untersuchungsergebnisse wie bspw. Bilder oder Röntgenaufnahmen automatisch zu speichern. In diesem Zusammenhang sind bereits Lösungen bekannt, welche es ermöglichen, die auf dem Rechner installierte Patientenverwaltungssoftware zumindest teilweise durch Bedienelemente des Behandlungs- oder Untersuchungsplatzes zu bedienen.

Eine Voraussetzung für das effiziente Nutzen der sich neuartig ergebenden, oben beschriebenen Möglichkeiten ist allerdings, dass die unterschiedlichen Komponenten in geeigneter Weise miteinander verbunden sind und in die Lage versetzt werden, in gewünschter Weise miteinander zu kommunizieren. Dies erfolgt im Rahmen eines sog. Praxisnetzwerks, also eines Kommunikationsnetzwerks, mit dem alle netzwerkfähigen Geräte verbunden sind, wobei eine Kommunikation sowohl drahtlos als auch über entsprechende Leitungen erfolgen kann. Voraussetzung hierfür ist allerdings, dass die netzwerkfähigen Komponenten in geeigneter Weise in das Netzwerk eingebunden werden, derart, dass sie eindeutig identifizierbar sind und in der gewünschten Weise mit den ihnen zugeordneten Komponenten kommunizieren können. Dieses Problem stellt sich insbesondere dann, wenn das Netzwerk bspw. mehrere Behandlungszimmer mit jeweils darin angeordneten Behandlungs- oder Untersuchungsplätzen aufweist und weiterhin innerhalb eines jeden Behandlungsraums ein Hinterkopf-PC mit darauf installierter Patientenverwaltungssoftware vorgesehen ist. In einem derartigen Fall sollte insbesondere eine primäre Verknüpfung zwischen den jeweils innerhalb eines Raums befindlichen Komponenten vorliegen, allerdings müssen diese ggf. auch mit weiteren, zentral genutzten Komponenten kommunizieren können.

In diesem Zusammenhang beschriebt die US 2011/145373 A1 ein Verfahren zum Konfigurieren der Kommunikation zwischen mehreren netzwerkfähigen Geräten innerhalb eines medizinischen Netzwerks. Hierbei wird eine Lösung vorgeschlagen, die eine Konfiguration der im Netz befindlichen medizinischen Ferngeräte von einem einzigen medizinischen Gerät aus, vorzugsweise von einem neu zu integrierenden Gerät aus, ermöglicht. Die Konfiguration mehrerer Geräte von einem einzigen Gerätestandort aus soll Zeit und Kosten für den gesamten Integrationsprozess sparen.

Es hat sich allerdings in der Vergangenheit gezeigt, dass das Konfigurieren derartiger Netzwerke allgemein für Personen ohne detaillierte IT-Kenntnisse nur schwer durchführbar ist. Bislang mussten also entsprechende Arbeiten in der Regel von Fachpersonal durchgeführt werden, was jedoch zu einem erhöhten Aufwand und reduzierter Flexibilität führte, da bei dem Hinzufügen eines neuen Geräts bzw. einer neuen Zuordnung einzelner Geräte untereinander jedes Mal Anpassungen durch das Fahrpersonal durchgeführt werden mussten.

Der vorliegenden Erfindung liegt dementsprechende die Aufgabenstellung zugrunde, eine neuartige Lösung anzubieten, welche es auch Personen ohne detaillierte IT-Kenntnisse oder Kenntnisse der Topologie des Praxisnetzwerks ermöglicht, dass Netzwerk einer zahnärztlichen Praxis in einfacher und komfortabler Weise zu konfigurieren.

Die Aufgabe wird durch ein Verfahren, welches die Merkmale des Anspruchs 1 aufweist, sowie durch ein zahnärztliches Praxisnetzwerk gemäß Anspruch 10 gelöst. Vorteilhafte Weiterbildungen der Erfindung sind Gegenstand der abhängigen Ansprüche.

Die erfindungsgemäße Lösung sieht vor, innerhalb des Netzwerks einen sog. Server-Provider-Service zur Verfügung zu stellen. Es handelt sich um einen Service, dessen Aufgabe darin besteht, unterschiedlichen Komponenten des Netzwerks, also bspw. einem Rechner, auf dem eine Patientensoftware installiert ist, oder einem zahnärztlichen Behandlungs- oder Untersuchungsplatz, Verbindungsdaten von Serverkomponenten des Netzwerks zur Verfügung zu stellen. Ferner ist als zentrale Komponente des Netzwerks ein sog. Equipment-Connector vorgesehen, der als zentraler und dauerhaft verfügbarer Service mit den Komponenten des Netzwerks kommuniziert, ihre logische Zuordnung verwaltet und die Komponenten über ihre Zuordnung informiert. Letztendlich stellt also der Equipment-Connector diejenige Komponente dar, welche für die gewünschte Kommunikation der verschiedenen Einheiten des Netzwerks untereinander verantwortlich ist, indem er sie zu Beginn in die Lage versetzt, in gewünschter Weise miteinander zu kommunizieren. Diese Komponenten, also bspw. der Rechner mit der Praxissoftware oder der zahnärztliche Behandlungs- oder Untersuchungsplatz sind dann erfindungsgemäß dazu ausgebildet, sich bei Inbetriebnahme mit dem Server-Provider-Service in Verbindung zu setzen und von diesem über die Kontaktierungsdaten des Equipment-Connectors informiert zu werden. Der Equipment-Connector wiederum kann dann mit den Komponenten kommunizieren und ihnen nach entsprechender Zuordnung zugehörige Informationen übermitteln.

Gemäß der vorliegenden Erfindung wird also ein Verfahren zum Konfigurieren des Netzwerks einer zahnärztlichen Praxis vorgeschlagen, wobei das Netzwerk zumindest zwei netzwerkfähige Einheiten, beispielsweise eine zahnärztliche Behandlungs- oder Untersuchungseinheit sowie einen von der Behandlungs- oder Untersuchungseinheit getrennten Hinterkopf-PC aufweist, und wobei das Verfahren folgende Schritte aufweist:
- Kontaktieren, durch die netzwerkfähigen Einheiten jeweils nach deren Inbetriebnahme, eines in dem Netzwerk befindlichen Server-Provider-Services,
- Übermitteln von dem Server-Provider-Service an die netzwerkfähigen Einheiten, von Kontaktierungsinformationen eines die Zuordnung von Geräten des Netzwerks verwaltenden und mit dem Netzwerk verbundenen Equipment-Connectors,
- Aufbau einer Kommunikation zwischen den netzwerkfähigen Einheiten und dem Equipment-Connector und individuelles Identifizieren der netzwerkfähigen Einheiten durch Equipment-Connector, wobei der Aufbau der Kommunikation mit Hilfe der von dem Server Provider Service übermittelten Kontaktierungsinformationen erfolgt;
- Festlegen von Zuordnungen der netzwerkfähigen Einheiten und Übermitteln von Zuordnungsinformationen durch den Equipment-Connector an die netzwerkfähigen Einheiten.

Die erfindungsgemäße Nutzung der beiden oben erwähnten neuartigen Dienste bzw. Instanzen, also des Server-Providers-Services und des Equipment-Connectors erlaubt es, dass das Netzwerk konfiguriert werden kann, ohne dass eine speziell hierfür ausgebildete Person den verschiedenen Einheiten bzw. Komponenten für die Kommunikation geeignete Adressen zuweisen muss. Stattdessen erlaubt es die erfindungsgemäße Lösung, dass sich die Komponenten selbstständig in dem Netzwerk anmelden und mit Hilfe des Server-Provider-Services und des Equipment-Connectors letztendlich in die Lage versetzt werden, grundsätzlich zu kommunizieren sowie insbesondere eine spezielle Kommunikation mit ihnen zugeordneten Einheiten aufzubauen. Ein besonders anschauliches und damit komfortables Zuordnen der verschiedenen Komponenten zueinander, also bspw. einer Behandlungs- oder Untersuchungseinheit zu einem Hinterkopf-PC kann dann dadurch erfolgen, dass - z.B. durch einen sich anmeldenden Hinterkopf-PC - eine Benutzeroberfläche, insbesondere eine grafische Benutzeroberfläche zur Verfügung gestellt wird, mit deren Hilfe eine logische Zuordnung der Geräte vorgenommen wird. Nachdem sich die verschiedenen Geräte in dem Netzwerk angemeldet haben, werden diese auf der Benutzeroberfläche anschaulich dargestellt und die für die Inbetriebnahme des Netzwerks verantwortliche Person muss lediglich noch eine entsprechende Zuordnung vornehmen, bspw. mit Hilfe einer einfachen sog. Drag-and-Drop-Aktion. Es handelt sich hierbei um Maßnahmen, die aufgrund ihrer Anschaulichkeit von jedermann durchgeführt werden können und insbesondere keine speziellen IT-Kenntnisse erfordern.

Das Kontaktieren des Server-Provider-Service durch die netzwerkfähigen Komponenten erfolgt dabei vorzugsweise jeweils mit Hilfe einer Broadcast-Nachricht. Hierdurch ist sichergestellt, dass erstmalig in Betrieb genommene Einheiten in einfacher Weise mit dem Server-Provider-Service in Kontakt treten können, ohne dass hierfür spezielle Maßnahmen durch einen Benutzer erforderlich sind.

Die Informationen bzgl. der Zuordnung verschiedener Komponenten des Netzwerks zueinander werden vorzugsweise in einem Speicher hinterlegt, der z.B. Bestandteil des Equipment-Connectors sein kann oder als separate Einheit, z.B. in Form einer externen Datenbank ausgebildet ist, welche dann mit dem Equipment-Connector verbunden ist. Der Equipment-Connector kann also jederzeit auf die entsprechenden Informationen zugreifen und insbesondere auch nach einem Aus- und erneuten Wiedereinschalten einzelner Komponenten diese unmittelbar darüber informieren, in welcher Weise sie in das Netzwerk eingebunden sind und mit welchen Einheiten sie kommunizieren können bzw. sollen.

Gemäß einer anderen vorteilhaften Weiterbildung sind der Server-Provider-Service und der Equipment-Connector zu einer funktionalen Einheit, die mit dem Netzwerk verbunden ist, zusammengefasst. Diese kann bspw. in Form eines separaten Rechners, der das gesamte Netzwerk verwaltet, ausgebildet sein. Denkbar wäre allerdings auch, dass der Server-Provider-Service und der Equipment-Connector Bestandteil eines sich innerhalb eines Behandlungsraums befindlichen Hinterkopf-PCs sind. Dieser Hinterkopf-PC ist dann nicht nur im Rahmen der üblichen Behandlung für die Nutzung mit einer Patientenverwaltungssoftware ausgelegt, sondern darüber hinaus auch für die Konfiguration und Verwaltung des Netzwerks verantwortlich. Darüber hinaus wäre es auch denkbar, z.B. aus Gründen einer optimierten Lastverteilung oder der technischen Verwaltung den Server-Provider-Service und den Equipment-Connector sowie ggf. die oben erwähnte Datenbank auf jeweils eigenen Maschinen bzw. Rechnern zu betreiben.

Gemäß einer anderen vorteilhaften Weiterbildung der Erfindung kann die Behandlungs- oder Untersuchungseinheit eine Display-Einheit zur Darstellung von Bedienoptionen aufweisen. Dabei kann insbesondere vorgesehen sein, dass diese Display-Einheit dazu ausgebildet ist, zumindest einen Teil der Bedienoptionen auf Basis von HTML-Dokumenten darzustellen. In diesem Fall ist dann ferner vorgesehen, dass innerhalb des Netzwerks zusätzlich zumindest ein HTTP-Server vorgesehen ist, der die HTML-Dokumente zur Verfügung stellt. Entsprechende Kontaktierungsinformationen werden dann wiederum automatisiert durch das Netzwerk, insbesondere durch den Server-Provider-Service oder den Equipment-Connector zur Verfügung gestellt.

Eine Darstellung der Bedienoptionen auf Basis von HTML-Dokumenten bringt dabei den Vorteil mit sich, dass die Behandlungs- oder Untersuchungseinheit bzw. das Display keine besondere Intelligenz aufweisen muss und insbesondere beim Hinzufügen und/oder Aktualisieren von Bedienoptionen nicht neu programmiert werden muss. Lediglich die von dem HTTP-Server zur Verfügung gestellten HTML-Dokumente müssen dann entsprechend angepasst werden. Dies bringt insbesondere Vorteile mit sich, wenn bspw. zusätzliche Geräte wie Röntgengeräte oder Intraoralkameras in das Netzwerk eingebunden werden und Optionen zum Ansteuern bzw. Bedienen dieser Geräte auch auf dem Display der Behandlungs- oder Untersuchungseinheit zur Verfügung gestellt werden sollen. Durch diese Maßnahme wird also die Flexibilität des gesamten Systems nochmals deutlich verbessert und es können jederzeit neue Geräte hinzugefügt werden.

Gemäß einer anderen Weiterbildung der Erfindung kann dabei vorgesehen sein, dass der Hinterkopf-PC mit mindestens einem weiteren Untersuchungs- oder Behandlungsgerät (wie bspw. einer Intraoralkamera, einem Laser oder dgl.) verbindbar ist, wobei dann die Verbindung vorzugsweise mittels USB erfolgt und der Hinterkopf-PC dazu ausgebildet ist, Informationen bzgl. des angeschlossenen Untersuchungs- oder Behandlungsgeräts an den Equipment-Connector zu übermitteln. Auch derartige Geräte, die also über den Hinterkopf-PC und damit indirekt dem Praxisnetzwerk hinzugefügt werden, werden also durch den Equipment-Connector erfasst, wodurch einerseits z.B. die Möglichkeit eröffnet wird, die Anordnung der verschiedenen Geräte in den unterschiedlichen Räumen der Praxis anschaulich darzustellen. Ferner wird wiederum die Möglichkeit eröffnet, dass diese zusätzlichen Geräte bspw. mit einer Behandlungs- oder Untersuchungseinheit gekoppelt und ggf. von dieser ferngesteuert werden.

Letztendlich erlaubt also die erfindungsgemäße Lösung, ein dentales Praxisnetzwerk, welches eine Vielzahl von Behandlungs- oder Untersuchungsgeräten und Rechner umfasst, in einfacher Weise zu konfigurieren und die sich aus der Verknüpfung der verschiedenen Geräte ergebenden Vorteile effizient nutzen zu können.

Nachfolgend soll die Erfindung anhand der beiliegenden Zeichnungen erläutert werden. Es zeigen:
- Figur 1: ein erstes Ausführungsbeispiel eines erfindungsgemäß ausgestalteten zahnärztlichen Praxisnetzwerks, welches gemäß der vorliegenden Erfindung konfiguriert wird;
- Figur 2a: ein denkbares Beispiel einer Nachricht, mit der eine aktivierte Komponente den Server-Provider-Service kontaktiert;
- Figur 2b: ein denkbares Beispiel einer Nachricht, mit der der Server-Provider-Service eine Netzwerk-Komponente über die IP-Adresse des Equipment-Connectors unterrichtet; und
- Figur 3: ein zweites Ausführungsbeispiel eines komplexeren erfindungsgemäßen zahnärztlichen Praxisnetzwerks.

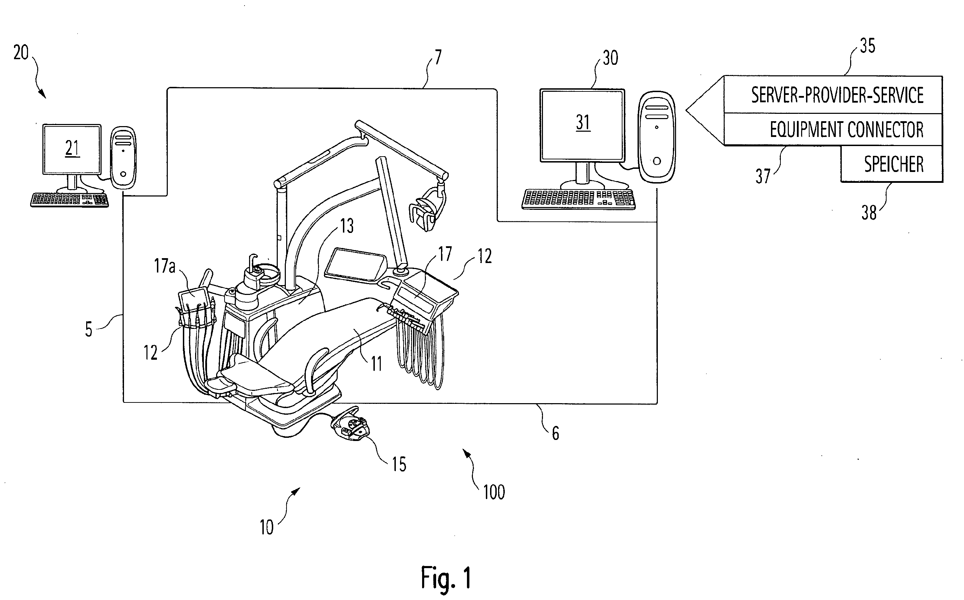

Figur 1 zeigt zunächst ein sehr einfach gehaltenes Ausführungsbeispiel eines Praxisnetzwerks, welche gemäß der vorliegenden Erfindung konfiguriert werden soll. Trotz der sehr geringen Komplexität des dargestellten Netzwerks kann anhand dieses Beispiels die erfindungsgemäße Vorgehensweise jedoch sehr anschaulich beschrieben werden.

Das allgemein mit dem Bezugszeichen 100 versehene Netzwerk besteht dabei primär aus einer zahnärztlichen Behandlungs- und Untersuchungseinheit 10 sowie einem sich üblicherweise in dem gleichen Raum, in dem sich auch die Behandlungs- oder Untersuchungseinheit 10 befindet, angeordneten PC 20. Die Behandlungs- und Untersuchungseinheit weist dabei in üblicher Weise einen Patientenstuhl 11 sowie daran angeordnete Mittel zur Behandlung und/oder Untersuchung eines Patienten auf. Bei diesen Mitteln kann es sich insbesondere um ein oder mehrere Halterungen 12 für zahnärztliche Behandlungsgeräte wie beispielsweise Sprayhandstück, Bohrhandstück, Zahnsteinentfernungsgerät oder dergleichen handeln. Die Versorgung dieser Geräte mit Strom sowie weiteren, für deren Nutzung erforderliche Medien wie beispielsweise Luft und/oder Wasser erfolgt mit Hilfe einer Versorgungseinheit 13, welche seitlich des Patientenstuhls 11 angeordnet ist. Die Steuerung des Patientenstuhls 11 erfolgt über einen Fußschalter 15, dessen einzelne Bedienelemente zumindest teilweise auch dazu benutzt werden können, Ansteuerungen eines aktuell genutzten Behandlungsinstruments vorzunehmen. Beispielsweise kann während der Behandlung mit Hilfe des Fußschalters 15 auch die Drehzahl eines Antriebs für den Bohrer eingestellt werden, wie dies bereits aus dem Stand der Technik hinlänglich bekannt ist.

Die Behandlungs- oder Untersuchungseinheit 10 kann weiterhin als Bestandteil des sog. Arzt-Tisches eine Display-Einheit 17 aufweisen, welche zur Darstellung beispielsweise von Betriebsparametern eines gerade genutzten Behandlungsinstruments genutzt wird. Auch anderweitige Patienteninformationen oder Informationen bezüglich der einzelnen Schritte einer durchzuführenden Behandlung können auf dem Display 17 dargestellt werden. Darüber hinaus können weitere Displays 17a vorhanden sein, welche ebenfalls der ergänzenden Darstellung von Informationen dienen.

Bei dem sich in der Nähe der Behandlungs- oder Untersuchungseinheit 10 befindlichen PC 20 handelt es sich um einen so genannten Hinterkopf-PC 20, der während der Durchführung einer Behandlung in der Regel zwar nicht unmittelbar genutzt wird, auf dem allerdings zusätzliche Informationen dargestellt werden können, welche für den Zahnarzt hilfreich sind. Hierzu ist auf dem PC 20 eine Patientenverwaltungssoftware installiert, mit deren Hilfe beispielsweise die Daten zu den verschiedenen Patienten verwaltet werden können. Diese Daten umfassen dabei nicht nur persönliche Daten des Patienten (wie z.B. dessen Alter, Adresse usw.) sondern auch Informationen hinsichtlich bereits durchgeführter oder noch durchzuführender Behandlungen und von Untersuchungsergebnissen. Insbesondere wäre es auch denkbar, dass im Zuge der Patientenverwaltung dem Patienten zugehörig Bildinformationen wie beispielsweise Röntgenbilder oder Bilder, welche mit Hilfe einer so genannten intraoralen Kamera erstellt worden, gespeichert werden. Auch diese Informationen können dann auf dem Bildschirm 21 des Hinterkopf-PCs 20 dargestellt und beispielsweise dazu genutzt werden, erforderliche Behandlungen dem Patienten in anschaulicher Weise zu erläutern.

Zwar könnten beide Einheiten, also die Behandlungs- oder Untersuchungseinheit 10 sowie der Hinterkopf-PC 20 durchaus unabhängig voneinander arbeiten, eine Kommunikation bzw. Zusammenarbeit zwischen beiden Einheiten 10, 20 wäre allerdings insofern wünschenswert, als hierdurch zusätzliche Vorteile erhalten werden können. Beispielsweise wäre eine Zusammenarbeit zwischen Hinterkopf-PC 20 und Behandlungs- oder Untersuchungseinheit 10 insofern hilfreich, als Daten, die durch die Patientenverwaltungssoftware verwaltet werden, auch auf dem Display 17 der Behandlungs- oder Untersuchungseinheit 10 dargestellt werden könnten. Für den Zahnarzt wäre dann also unmittelbar ersichtlich, um welchen Patienten es sich handelt und welche Aspekte bei der Untersuchung oder Behandlung gegebenenfalls zu berücksichtigen sind. In umgekehrter Weise könnte durch eine Verbindung zwischen Behandlungs- und Untersuchungseinheit 10 und Hinterkopf-PC 20 sichergestellt werden, dass durchgeführte Behandlungen automatisch durch die Patientenverwaltungssoftware des Hinterkopf-PCs 20 protokolliert werden. Schließlich wäre es auch denkbar, dass im Falle einer Verbindung zwischen Behandlungs- oder Untersuchungseinheit 10 und Hinterkopf-PC 20 Bedienelemente der Behandlungs- oder Untersuchungseinheit 10 zur Steuerung des Hinterkopf-PCs 20 genutzt werden könnten. So wäre beispielsweise denkbar, dass mit Hilfe des Fußschalters 15 eine Steuerung der an dem Hinterkopf-PC 20 laufenden Patientenverwaltungssoftware erfolgt. Insbesondere wäre in diesem Zusammenhang beispielsweise denkbar, dass mit Hilfe des Fußschalters 15 die bereits zuvor erwähnten Röntgenbilder oder anderweitige Untersuchungsergebnisse wahlweise auf dem Display des Hinterkopf-PCs 20 aufgerufen werden können, ohne dass eine Betätigung der Tastatur des PCs 20 erforderlich ist, was wiederum zu Vorteilen hinsichtlich der in einer zahnärztlichen Praxis einzuhaltenden Hygienevorschriften führt.

Eine derartige Zusammenarbeit ist allerdings nur dann möglich, wenn beide Geräte, also Behandlungs- oder Untersuchungseinheit 10 und Hinterkopf-PC 20 in die Lage versetzt werden, miteinander zu kommunizieren. Dies erfordert nicht nur eine physische Verbindung beispielsweise mit Hilfe des dargestellten LAN-Kabels 5 sondern es ist darüber hinaus auch erforderlich, dass beide Einheiten 10, 20 innerhalb des hierdurch gebildeten Netzwerks 100 in die Lage versetzt werden, miteinander Informationen auszutauschen. Das heißt, sie müssen in die Lage versetzt werden, Daten derart über das Netzwerk 100 zu versenden, dass diese auch tatsächlich von der adressierten Einheit empfangen und in gewünschter Weise umgesetzt werden können. Mit Hilfe der nachfolgend näher beschriebenen Erfindung soll dieses Problem gelöst werden.

Die erfindungsgemäße Lösung beruht auf dem Gedanken, dass nicht nur die einzelnen Komponenten mit dem Netzwerk 100 verbunden werden, sondern in dem Netzwerk 100 zusätzliche Komponenten bzw. Services zur Verfügung stehen, durch welche die Konfiguration insgesamt erleichtert wird. Im dargestellten Ausführungsbeispiel sind diese zusätzlichen Funktionen bzw. Services in Form eines weiteren, das Netzwerk 100 verwaltenden PCs 30 realisiert. Dieser ist - wie schematisch dargestellt - über Verbindungen 6 bzw. 7 wiederum mit den beiden Netzwerkkomponenten, also Behandlungs- oder Untersuchungseinheit 10 und Hinterkopf-PC 20 verbunden, wobei darauf hinzuweisen ist, dass die Erfindung nicht auf eine bestimmte Netzwerktopologie beschränkt ist. In der Regel weist das Netzwerk 100 dann einen - aus Gründen der Übersichtlichkeit nicht dargestellten - zentralen Switch auf, mit dem jede Netzwerkkomponente verbunden ist. Denkbar wären allerdings auch andere Strukturen wie z.B. Ring-Topologien, sofern diese eine geeignete Kommunikation der Netzwerkkomponenten ermöglichen. Ferner wäre auch denkbar, dass in dem Netzwerk 100 insgesamt eine drahtlose Kommunikation erfolgt.

Wesentlich ist, dass dieser zusätzliche PC 30 zwei verschiedene Komponenten bzw. Services zur Verfügung stellt, einerseits einen so genannten Server-Provider-Service 35 sowie andererseits einen so genannten Equipment-Connector 37. Beide Komponenten sind im dargestellten Fall softwaremäßig auf diesem separaten PC 30 implementiert und dienen der Unterstützung bei der Konfiguration und Verwaltung des Netzwerks 100 insgesamt. Wie später noch näher erläutert wird, können allerdings Server-Provider-Service 35 und Equipment-Connector 37 auch in anderer Weise innerhalb des Netzwerks betrieben werden.

Dabei besteht die primäre Aufgabe des Service-Provider-Services 35 darin, dass dieser den verschiedenen Teilnehmern des Netzwerks 100 Verbindungsdaten von weiteren Serverkomponenten des Netzwerks 100 zur Verfügung stellt. Der Equipment-Connector 37 wiederum stellt einen zentralen, dauerhaft verfügbaren Service des Netzwerks 100 dar, der den zentralen Kommunikationspunkt für alle verbundenen Einheiten bildet, für die Zuordnung der Einheiten untereinander verantwortlich ist und die Einheiten auch entsprechend hierüber informiert. Des Weiteren ist der Equipment-Connector 37 mit einem Speicher 38 ausgestattet bzw. mit einem entsprechenden Speicher 38 verbunden, in dem die Konfigurationsinformationen bezüglich des Netzwerks 100 hinterlegt sind.

Die Vorgehensweise bei der Inbetriebnahme bzw. Konfiguration des Netzwerks 100 ist nunmehr wie folgt.

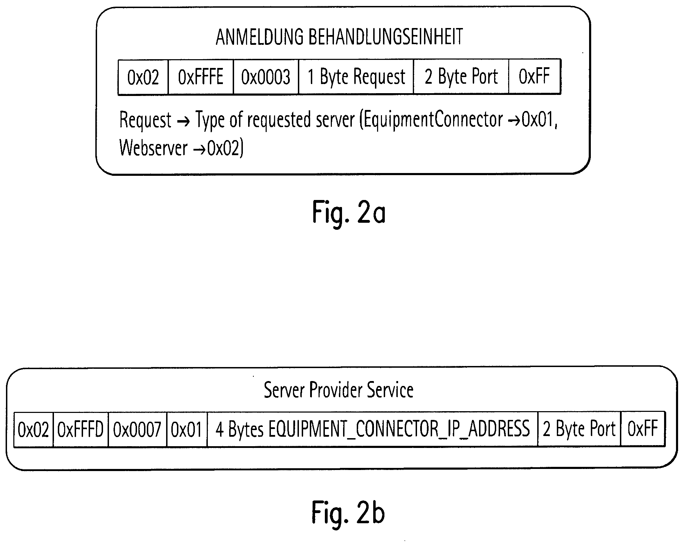

Jede Netzwerkkomponente, also beispielsweise die Behandlungs- oder Untersuchungseinheit 10 oder der Hinterkopf-PC 20 versucht bei Inbetriebnahme, also bei Einschalten zunächst für die nachfolgende Anmeldung in dem Netzwerk 100 erforderliche Informationen abzurufen. Dies kann in einfacher Weise dadurch erfolgen, dass die entsprechende Netzwerkkomponente mittels einer Broadcast-Nachricht den Server-Provider-Service 35 kontaktiert, wobei diese entsprechende Nachricht gemäß einem vorgeschriebenen Protokoll aufgebaut ist. Ein denkbares Ausführungsbeispiel einer entsprechenden Anfrage ist hierbei in Figur 2a dargestellt, wobei diese Anfrage insbesondere einen Bestandteil enthält, der die sich anmeldende Netzwerkkomponente identifiziert. Es kann sich hierbei einerseits um Daten handeln, welche den Typ der Komponente (also beispielsweise Hinterkopf-PC oder Untersuchungs- oder Behandlungseinheit) charakterisieren. Darüber hinaus enthält die Information vorzugsweise auch eine eindeutige Kennung der Netzwerkkomponente, z.B. eine während der Herstellung vergebene Seriennummer, da - wie nachfolgend noch näher erläutert - durchaus der Fall auftreten kann, dass sich mehrere gleichartige Komponenten in dem Netzwerk 100 anmelden.

Diese Broadcastinformation wird von dem Server-Provider-Service 35 erkannt, der sie als eine Anfrage von Verbindungsdaten hinsichtlich der gewünschten Serverkomponente(n) interpretiert. Eine Rückmeldung erfolgt dann dahingehend, dass die sich meldende Serverkomponente von dem Server-Provider-Service 35 - nunmehr individuell - darüber informiert wird, unter welcher Adresse insbesondere der Equipment-Connector 37 innerhalb des Netzwerks 100 kontaktierbar ist. Dies kann z.B. über eine Rückmeldung erfolgen, wie sie schematisch in Figur 2b dargestellt ist.

Nach Austausch der entsprechenden Informationen ist also der Netzwerkkomponente insbesondere die Adresse bekannt, unter welcher sie den Equipment-Connector 37 kontaktieren und mit diesem kommunizieren kann. Dies wiederum ermöglicht den Aufbau einer Kommunikation zwischen Netzwerkkomponente und Equipment-Connector 37, in deren Rahmen dann die eigentliche Anmeldung des Geräts in dem Netzwerk 100 stattfindet. Erst der Equipment-Connector 37 behandelt diese Verbindung "stateful" und identifiziert die verschiedenen Geräte notwendigerweise individuell.

Als Ergebnis dieser Schritte insgesamt ist dann also dem Equipment-Connector 37 bekannt, welche verschiedenen Komponenten mit dem Netzwerk 100 verbunden sind und unter welcher IP Adresse diese kontaktiert werden können.

In einem weiteren Schritt wird dann eine logische Zuordnung zwischen Behandlungs- oder Untersuchungseinheit 10 und Hinterkopf-PC vorgenommen. Anschließend werden beide Netzwerkkomponenten von dem Equipment-Connector 37 über die entsprechende Zuordnung informiert und hierdurch in die Lage versetzt, miteinander zu kommunizieren, um die oben erwähnte vorteilhafte Zusammenarbeit zwischen beiden Einheiten 10, 20 umzusetzen. Dabei kann sowohl eine unmittelbare Kommunikation zwischen Behandlungs- oder Untersuchungseinheit 10 und Hinterkopf-PC 20 vorliegen als auch eine Kommunikation, die über den Equipment-Connector 37 verwaltet wird, wobei unabhängig davon die Daten hinsichtlich der Zuordnung der verschiedenen Netzwerkkomponenten zueinander in dem Speicher 38 hinterlegt werden. Eine unmittelbare bzw. 1:1-Verbindung zwischen Behandlungs- oder Untersuchungseinheit 10 und zugehörigem Hinterkopf-PC 20 bringt dabei den Vorteil mit sich, dass die Zusammenarbeit zwischen beiden Geräten 10, 20 auch weiterhin genutzt werden kann, wenn insgesamt eine Störung des Netzwerks 100 oder zum Beispiels ein vorübergehender Ausfall des Equipment-Connectors 37 vorliegt. Die Betriebssicherheit der Anordnung wird durch diese Maßnahme also erhöht.

Eine Zuordnung zwischen den verschiedenen Netzwerkkomponenten 10 und 20 erfolgt dabei vorzugsweise mit Hilfe einer grafischen Benutzeroberfläche, die z.B. auf dem Bildschirm 21 des Hinterkopf-PCs 20 durch eine darauf installierte Software, die vorzugsweise einen Bestandteil der Patientenverwaltungssoftware darstellt, zur Verfügung gestellt werden kann. Auf dieser Benutzeroberfläche können beispielsweise die sich in dem Netzwerk 100 anmeldenden Komponenten graphisch dargestellt werden. Im Rahmen einer entsprechenden Eingabe durch einen Benutzer, beispielsweise durch eine so genannte Drag-and-Drop-Aktion kann dann eine Zuordnung zu verschiedenen Behandlungsräumen und/oder anderen Geräten vorgenommen werden. Alternativ könnte die Benutzeroberfläche auch auf dem Bildschirm 31 des PCs 30 zur Verfügung gestellt werden oder es könnte auch eine durch den Equipment-Connector 37 automatisierte Zuordnung erfolgen, die beispielsweise abhängig von der Reihenfolge, in der die Komponenten eingeschaltet werden und sich dann wie oben beschrieben in dem Netzwerk 100 anmelden, vorgenommen wird.

Die Zuordnungsinformationen werden wie bereits erwähnt in dem Speicher 38 hinterlegt und stehen dann während des späteren Betriebs dauerhaft zur Verfügung. Nach einem Aus- und wieder Einschalten einzelner Komponenten wiederholt sich hierbei der oben beschriebene Vorgang, wobei der Equipment-Connector 37 dann jedoch unmittelbar die Informationen hinsichtlich der Zuordnung der Netzwerkkomponenten untereinander zur Verfügung stellen kann und nicht erst durch einen Benutzer eine erneute Zuordnung vorgenommen werden muss.

Wesentlich ist, dass bei dem erfindungsgemäßen Verfahren das Anmeldung und Zuweisen der für die Kommunikation erforderlichen Informationen zu den verschiedenen Netzwerkkomponenten weitestgehend automatisiert abläuft und ein Benutzer lediglich gegebenenfalls eine Zuordnung der Komponenten zueinander vornehmen muss. Er muss jedoch nicht mehr den Komponenten einzelne Parameter wie beispielsweise IP-Adressen oder dergleichen zuweisen, wodurch der Aufwand für den Benutzer deutlich reduziert wird und insbesondere die Konfiguration auch ohne detaillierte Kenntnisse durchgeführt werden kann. Nachdem alle beteiligten Geräte in die Lage versetzt wurden, zu kommunizieren, kann dann ggf. auch eine verschlüsselte Kommunikation stattfinden, wofür die Geräte zunächst untereinander entsprechende Schlüssel austauschen.

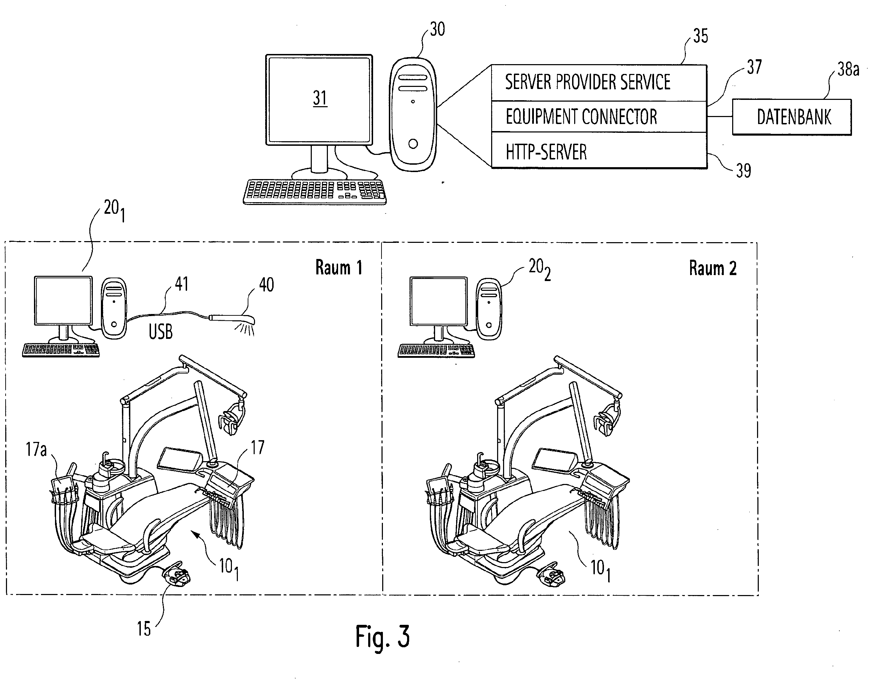

Das zuvor beschriebene erfindungsgemäße Konzept kann in gleicher Weise auch zur Konfigurierung deutlich komplexerer Netzwerke genutzt werden, wie das weitere Ausführungsbeispiel, welches in Figur 3 dargestellt ist, zeigt.

In diesem Fall wird davon ausgegangen, dass die zahnärztliche Praxis zwei Behandlungsräume aufweist, in denen jeweils eine Behandlungs- oder Untersuchungseinheit 10₁ und 10₂ sowie ein Hinterkopf-PC 20₁ bzw. 20₂ angeordnet sind. Auf die Darstellung der LAN-Kabel wurde in diesem Fall aus Gründen der Übersichtlichkeit verzichtet, wobei - wie bereits erwähnt - auch eine drahtlose Kommunikation zwischen den verschiedenen Geräten denkbar wäre bzw. eine beliebige Netzwerktopologie vorliegen kann.

In diesem zweiten Ausführungsbeispiel ist das Konfigurieren von wesentlicher Bedeutung, da bei der erstmaligen Inbetriebnahme der verschiedenen Netzwerkkomponenten diesen unbekannt ist, in welchem Raum sie sich befinden und mit welcher weiteren Komponente sie eine Kombination bilden sollen. Der Hinterkopf-PC 20₁ soll also eindeutig der entsprechenden Behandlungs- oder Untersuchungseinheit 10₁, die sich im gleichen Raum befindet, zugeordnet werden. Gleiches gilt für die beiden in dem zweiten Raum befindlichen Komponenten 10₂ und 20₂.

Die Vorgehensweise zum Konfigurieren des Netzwerks 100 ist allerdings wiederum die gleiche. Das heißt, auch bei einem komplexeren System kontaktiert jede Netzwerkkomponente zunächst nach ihrer Inbetriebnahme den Server-Provider-Service 35 im Rahmen der oben beschriebenen Broadcast-Meldung, um Verbindungsnahten der gewünschten Serverkomponenten, insbesondere des Equipment-Connectors 37 zu erhalten. Im Rahmen der bereits erwähnten grafischen Benutzeroberfläche kann dann zum Beispiel der Zahnarzt jede sich identifizierende Netzwerkkomponente einerseits einem der Behandlungsräume zuordnen sowie andererseits festlegen, in welcher Weise eine Zuordnung zu einem weiteren Gerät erfolgen soll. Auch in diesem Fall sind dann nach Abschluss des Verfahrens allen Komponenten die jeweils zur Kommunikation erforderlichen IP-Adressen bekannt und die Kommunikation kann mittels TCP/IP erfolgen.

Eine Erweiterung der bislang beschriebenen Gedanken, die allerdings in gleicher Weise auch bei dem Ausführungsbeispiel gemäß Figur 1 zum Einsatz kommen könnte, besteht im dargestellten Ausführungsbeispiel darin, dass Bedienoptionen für die Behandlungs- oder Untersuchungseinheit 10 sowie den PC 20 auf dem Display 17 der Behandlungs- oder Untersuchungseinheit 20 mit Hilfe von HTML-Dokumenten dargestellt werden. Diese Vorgehensweise ist insofern von Vorteil, als damit äußerst flexibel das Display 17 der Behandlungs- oder Untersuchungseinheit genutzt werden kann, ohne dass hierfür eine spezielle Software zu programmieren ist. Für den Fall, dass - wie nachfolgend noch näher beschrieben - ein neues Gerät in das System integriert wird, welches beispielsweise über den Fußschalter 15 angesteuert werden soll, muss lediglich ein entsprechend zugehöriges HTML-Dokument abgerufen und auf dem Display 17 dargestellt werden. Zur Durchführung dieser Maßnahmen ist lediglich erforderlich, dass die Untersuchungs- oder Behandlungseinheit 10 bzw. das zugehörige Display 17 mit einem Web-Browser ausgestattet ist, der die Darstellung der HTML-Seiten ermöglicht.

Im dargestellten Ausführungsbeispiel ist weiterhin vorgesehen, dass die HTML-Seiten von einem entsprechenden HTTP-Server 38 zur Verfügung gestellt werden, der wiederum in dem zentralen Rechner 30 implementiert ist, der auch den Server-Provider-Service 35 sowie den Equipment-Connector 37 zur Verfügung stellt. Da die Behandlungs- oder Untersuchungseinheit 10 bzw. das Display 17 (sofern dies - was durchaus denkbar wäre - eine eigenständige Komponente innerhalb des Netzwerks 100 darstellt) zum Abrufen von HTML-Seiten mit dem HTTP-Server 39 kommunizieren können muss, muss den entsprechenden Komponenten wiederum die IP-Adresse des HTTP-Servers 39 mitgeteilt werden, was in gleicher Weise durch eine Nachricht entsprechend dem oben beschriebenen Protokoll erfolgt. Die entsprechende Nachricht, die analog zu der in Figur 2b dargestellten Nachricht aufgebaut ist, kann dabei entweder durch den Server-Provider-Service 35 oder vorzugsweise durch den Equipment-Connector 37 übermittelt werden.

Die Darstellung von Bedienoptionen mittels HTML-Dokumenten bringt dabei insbesondere dann Vorteile mit sich, wenn hierdurch zusätzliche Geräte angesteuert werden sollen. Soll beispielsweise im Raum 1 eine Intraoralkamera 40 genutzt und mit Hilfe von Bedienelementen des Untersuchungs- oder Behandlungsplatzes 10 gesteuert werden, so muss einfach durch den HTTP-Server 39 eine entsprechende HTML-Seite zur Verfügung gestellt werden, die dann auf dem Display 17 dargestellt wird und Bedienoptionen hinsichtlich der Kamerasteuerung beinhaltet. Die Information, dass eine Kamera 40 vorhanden ist, kann dabei abhängig davon, mit welcher Einheit die Kamera 40 verbunden wird, entweder durch die Behandlungs- oder Untersuchungseinheit 10 bzw. den Hinterkopf-PC 20 dem Equipment-Connector 37 und/oder dem HTTP-Server 39 mitgeteilt werden. Eine bevorzugte Vorgehensweise würde beispielsweise darin bestehen, dass die Kamera 40 - wie schematisch dargestellt - mittels eines USB-Kabels 41 an den Hinterkopf-PC 20₁ angeschlossen wird. Dieser erkennt das Vorhandensein des zusätzlichen Geräts und teilt dies insbesondere dem Equipment-Connector 37 mit, der wiederum den HTTP-Server 39 dazu veranlasst, ein entsprechendes HTML-Dokument an das Display 17 der Untersuchungs- oder Behandlungseinheit 10 zu übermitteln. Auf der Benutzeroberfläche des Displays 17 wird dann entweder eine vollständig neue, die Steuerung der Kamera 40 ermöglichende Oberfläche dargestellt oder es erscheint ein zusätzliches Symbol, mit dem eine Kamerasteuerung aufgerufen werden kann. Ein Vorteil dieser Vorgehensweise besteht dabei darin, dass der Equipment-Connector 37 sämtliche Einheiten der Praxis verwalten kann und beispielsweise deren räumliche Anordnung und Zuordnung zu verschiedenen Geräten untereinander grafisch darstellen kann. Hierdurch ist grundsätzlich die Information vorhanden, welche Geräte sich in welchen Räumlichkeiten befinden. Dies gilt - wie anhand des Beispiels erläutert - nicht nur für Komponenten, die unmittelbar mit dem Netzwerk 100 verbunden sind, sondern auch für zusätzliche Geräte, die beispielsweise über eine USB-Verbindung mit einer Netzwerkkomponente verbunden werden.

Selbstverständlich können allerdings auch weitere Geräte unmittelbar mit Netzwerk 100 verbunden werden, was sich z.B. für den Fall einer Röntgeneinrichtung anbieten würde. Auch diese netzwerkfähigen Geräte verhalten sich dann in gleicher Weise wie auch z.B. der Hinterkopf-PC 20 oder die Behandlungs- oder Untersuchungseinheit 10, um sich in dem Netzwerk 100 anzumelden und die zur Kommunikation erforderlichen Informationen mitzuteilen bzw. abzurufen.

Bezüglich der oben erwähnten Darstellung von Bedienoptionen mittels HTML-Dokumenten ist ferner anzumerken, dass eine weitere Besonderheit dieser Vorgehensweise darin bestehen kann, dass nicht nur durch die Displayeinheit 17 bzw. die Behandlungs- oder Untersuchungseinheit 10 das Abrufen neuer HTML-Dokumente initiiert werden kann, sondern auch der HTTP-Server 39 von sich aus eine Aktualisierung vornehmen kann. Dies kann erforderlich sein, wenn neue Geräte in das System integriert werden und die Information hierzu der Untersuchungs- oder Behandlungseinheit 10 nicht unmittelbar vorliegt, wie dies beispielsweise beim Anschließen der Intraoralkamera 40 an den Hinterkopf-PC 20 der Fall ist.

Letztendlich erlaubt also die erfindungsgemäße Vorgehensweise, alle Komponenten eines zahnärztlichen Praxisnetzwerks in einfacher Weise zu konfigurieren und logisch einander zuzuordnen. Darüber hinaus können auch zusätzliche, mit einzelnen Netzwerkkomponenten verbundene Geräte erkannt und durch den Equipment-Connector verwaltet werden, wodurch der Gesamtkomfort und die Übersicht bei der Nutzung der verschiedenen Geräte deutlich erhöht wird.

Dabei kann alternativ zu den zwei zuvor beschriebenen Ausführungsbeispielen auch vorgesehen sein, dass der Equipment-Connector die entsprechend erfassten Daten nicht in einem internen Speicher ablegt, sondern - wie in Figur 3 gezeigt - mit einer externen Datenbank 38a verbunden ist. Auch müssen Server-Provider-Service und Equipment-Connector sowie HTTP-Server nicht wie dargestellt in einem separaten Gerät implementiert werden, sondern könnten insbesondere auch auf einem der Hinterkopf-PCs und/oder auch jeweils separat auf unterschiedlichen PCs bzw. Maschinen des Netzwerks realisiert werden. Ein Betreiben der verschiedenen Services auf jeweils separaten Geräten kann dabei dann von Vorteil sein, wenn hierdurch eine bessere Lastverteilung erzielt oder die technische Verwaltung optimiert werden kann. Wesentlich ist jedoch, dass insbesondere der Server-Provider-Service im gesamten Netzwerk lediglich ein einziges Mal zur Verfügung steht, um ein eindeutiges Anmelden der einzelnen Komponenten und das entsprechende Zuordnen der Informationen hinsichtlich der Kommunikation sicherzustellen.

## Patentansprüche

1. Verfahren zum Konfigurieren des Netzwerks (100) einer zahnärztlichen Praxis, wobei das Netzwerk (100) zumindest zwei netzwerkfähigen Einheiten, beispielsweise eine zahnärztliche Behandlungs- oder Untersuchungseinheit (10) sowie einen von der Behandlungs- oder Untersuchungseinheit (10) getrennten Hinterkopf-PC (20) aufweist, und wobei das Verfahren folgende Schritte aufweist:
• Kontaktieren, durch die netzwerkfähigen Einheiten jeweils nach deren Inbetriebnahme, eines in dem Netzwerk befindlichen Server Provider Services (35),
• Übermitteln, von dem Server Provider Service (35) an die netzwerkfähigen Einheiten, von Kontaktierungsinformationen eines die Zuordnung von Geräten des Netzwerks (100) verwaltenden und mit dem Netzwerk (100) verbundenen Equipment-Connectors (37),
• Aufbau einer Kommunikation zwischen den netzwerkfähigen Einheiten und dem Equipment-Connector (37) und individuelles Identifizieren der netzwerkfähigen Einheiten durch Equipment-Connector (37), wobei der Aufbau der Kommunikation mit Hilfe der von dem Server Provider Service (35) übermittelten Kontaktierungsinformationen erfolgt;
• Festlegen von Zuordnungen der netzwerkfähigen Einheiten und Übermitteln von Zuordnungsinformationen durch den Equipment-Connector (37) an die netzwerkfähigen Einheiten.

2. Verfahren nach Anspruch 1,
**dadurch gekennzeichnet,**
**dass** das Kontaktieren des Server Provider Services (35) durch die netzwerkfähigen Einheiten jeweils mit Hilfe einer Broadcast-Nachricht erfolgt.

3. Verfahren nach Anspruch 1 oder 2,
**dadurch gekennzeichnet,**
**dass** das Zuordnen der netzwerkfähigen Einheiten mit Hilfe einer Benutzeroberfläche, insbesondere einer graphischen Benutzeroberfläche erfolgt, wobei die Benutzeroberfläche vorzugsweise durch den Hinterkopf-PC (20) zur Verfügung gestellt wird.

4. Verfahren nach einem der vorherigen Ansprüche,
**dadurch gekennzeichnet,**
**dass** die Zuordnungsinformationen in einem Speicher (38) hinterlegt werden, der Bestandteil des Equipment-Connectors (37) ist oder mit diesem verbunden ist.

5. Verfahren nach einem der vorherigen Ansprüche,
**dadurch gekennzeichnet,**
**dass** der Server Provider Service (35) und der Equipment-Connector (37) in einem Hinterkopf-PC (20) implementiert sind.

6. Verfahren nach einem der vorherigen Ansprüche,
**dadurch gekennzeichnet,**
**dass** die Behandlungs- oder Untersuchungseinheit (10) eine Display-Einheit (17) zur Darstellung von Bedienoptionen aufweist.

7. Verfahren nach Anspruch 6,
**dadurch gekennzeichnet,**
**dass** die Display-Einheit (17) dazu ausgebildet ist, zumindest einen Teil der Bedienoptionen auf Basis von HTML-Dokumenten darzustellen, wobei das Verfahren zusätzlich folgenden Schritt umfasst:
Übermitteln, an die Behandlungs- oder Untersuchungseinheit (10) oder die Display-Einheit (17) von Kontaktierungsinformationen eines HTTP-Servers (39), der zur Ausgabe der HTML-Dokumente ausgebildet ist.

8. Verfahren nach Anspruch 7,
**dadurch gekennzeichnet,**
**dass** die Übermittlung der Kontaktierungsinformationen des HTTP-Servers (39) durch den Server Provider Service (35) oder den Equipment-Connector (37) erfolgt.

9. Verfahren nach einem der vorherigen Ansprüche,
**dadurch gekennzeichnet,**
**dass** der Hinterkopf-PC (20) mit mindestens einem weiteren Untersuchungs- oder Behandlungsgerät, beispielsweise einer Intraoralkamera (40) verbindbar ist, wobei die Verbindung vorzugsweise über USB erfolgt,
und wobei der Hinterkopf-PC (20) dazu ausgebildet ist, Informationen bzgl. eines angeschlossenen Untersuchungs- oder Behandlungsgeräts an den Equipment-Connector (37) zu übermitteln.

10. Zahnärztliches Praxisnetzwerk (100) mit zumindest zwei netzwerkfähigen Einheiten, beispielsweise einer zahnärztlichen Behandlungs- oder Untersuchungseinheit (10) und einen von der Behandlungs- oder Untersuchungseinheit (10) getrennten Hinterkopf-PC (20), einem Server Provider Service (35) sowie einem Equipment-Connector (37),
wobei die netzwerkfähigen Einheiten jeweils dazu ausgebildet sind, nach ihrer Inbetriebnahme den Server Provider Service (35) zu kontaktieren,
wobei der Server Provider Service (35) dazu ausgebildet ist, an die netzwerkfähigen Einheiten Kontaktierungsinformationen des die Zuordnung von Geräten des Netzwerks (100) verwaltenden Equipment-Connectors (37) zu übermitteln,
wobei die netzwerkfähigen Einheiten ferner jeweils dazu ausgebildet sind, mit Hilfe der von dem Server Provider Service (35) übermittelten Kontaktierungsinformationen eine Kommunikation mit dem Equipment-Connector (37) aufzubauen und durch diesen individuell identifiziert zu werden;
und wobei der Equipment-Connector (37) dazu ausgebildet ist, Zuordnungsinformationen an die netzwerkfähigen Einheiten zu übermitteln.

11. Zahnärztliches Praxisnetzwerk nach Anspruch 10,
**dadurch gekennzeichnet,**
**dass** die netzwerkfähigen Einheiten dazu ausgebildet sind, den Server Provider Service (35) mit Hilfe einer Broadcast-Nachricht zu kontaktieren.

12. Zahnärztliches Praxisnetzwerk nach Anspruch 10 oder 11,
**dadurch gekennzeichnet,**
**dass** dieses eine Benutzeroberfläche, insbesondere eine graphische Benutzeroberfläche aufweist, welche vorzugsweise durch den Hinterkopf-PC (20) zur Verfügung gestellt wird und mit deren Hilfe eine logisch Zuordnung der netzwerkfähigen Einheiten vorgenommen werden kann.

13. Zahnärztliches Praxisnetzwerk nach einem der Ansprüche 10 bis 12,
**dadurch gekennzeichnet,**
**dass** dieses einen Speicher zum Hinterlegen der Zuordnungsinformationen aufweist, der Bestandteil des Equipment-Connectors (37) ist oder mit diesem verbunden ist.

14. Zahnärztliches Praxisnetzwerk nach einem der Ansprüche 10 bis 13,
**dadurch gekennzeichnet,**
**dass** der Server Provider Service (35) und der Equipment-Connector (37) in einem Hinterkopf-PC (20) implementiert sind.

15. Zahnärztliches Praxisnetzwerk nach einem der Ansprüche 10 bis 14,
**dadurch gekennzeichnet,**
**dass** die Behandlungs- oder Untersuchungseinheit (10) eine Display-Einheit (17) zur Darstellung von Bedienoptionen aufweist.

16. Zahnärztliches Praxisnetzwerk nach Anspruch 15,
**dadurch gekennzeichnet,**
**dass** die Display-Einheit (17) dazu ausgebildet ist, zumindest einen Teil der Bedienoptionen auf Basis von HTML-Dokumenten darzustellen,
wobei das Netzwerk (100) zusätzlich einen HTTP-Server (39) zur Ausgabe der HTML-Dokumente aufweist.

17. Zahnärztliches Praxisnetzwerk nach einem der Ansprüche 10 bis 16,
**dadurch gekennzeichnet,**
**dass** der Hinterkopf-PC (20) mit mindestens einem weiteren Untersuchungs- oder Behandlungsgerät, beispielsweise einer Intraoralkamera (40) verbindbar ist, wobei die Verbindung vorzugsweise über USB erfolgt,
und wobei der Hinterkopf-PC (20) dazu ausgebildet ist, Informationen bzgl. eines angeschlossenen Untersuchungs- oder Behandlungsgeräts an den Equipment-Connector (37) zu übermitteln.

## Claims

1. Method for configuring the network (100) in a dental practice, wherein the network (100) has at least two network-capable units, for example a dental treatment or examination unit (10) and a behind-the-head PC (20) which is separate from the treatment or examination unit (10), and wherein the method has the following steps of:
• contacting a server provider service (35) in the network by means of the network-capable units after they have been started up in each case,
• transmitting, from the server provider service (35) to the network-capable units, contact information relating to an equipment connector (37) which manages the assignment of devices in the network (100) and is connected to the network (100),
• setting up communication between the network-capable units and the equipment connector (37) and individually identifying the network-capable units by means of the equipment connector (37), wherein the communication is set up with the aid of the contact information transmitted by the server provider service (35);
• determining assignments of the network-capable units and transmitting assignment information to the network-capable units by means of the equipment connector (37).

2. Method according to Claim 1,
**characterized**
**in that** the server provider service (35) is contacted by the network-capable units in each case with the aid of a broadcast message.

3. Method according to Claim 1 or 2,
**characterized**
**in that** the network-capable units are assigned with the aid of a user interface, in particular a graphical user interface, wherein the user interface is preferably provided by the behind-the-head PC (20).

4. Method according to one of the preceding claims,
**characterized**
**in that** the assignment information is stored in a memory (38) which is part of the equipment connector (37) or is connected to the latter.

5. Method according to one of the preceding claims,
**characterized**
**in that** the server provider service (35) and the equipment connector (37) are implemented in a behind-the-head PC (20).

6. Method according to one of the preceding claims,
**characterized**
**in that** the treatment or examination unit (10) has a display unit (17) for displaying operating options.

7. Method according to Claim 6,
**characterized**
**in that** the display unit (17) is designed to display at least some of the operating options on the basis of HTML documents, wherein the method additionally comprises the following step of:
transmitting, to the treatment or examination unit (10) or to the display unit (17), contact information relating to a HTTP server (39) which is designed to output the HTML documents.

8. Method according to Claim 7,
**characterized**
**in that** the contact information relating to the HTTP server (39) is transmitted by the server provider service (35) or the equipment connector (37).

9. Method according to one of the preceding claims,
**characterized**
**in that** the behind-the-head PC (20) can be connected to at least one further examination or treatment device, for example an intraoral camera (40), wherein the connection is preferably effected via USB,
and wherein the behind-the-head PC (20) is designed to transmit information relating to a connected examination or treatment device to the equipment connector (37).

10. Dental practice network (100) having at least two network-capable units, for example a dental treatment or examination unit (10) and a behind-the-head PC (20) which is separate from the treatment or examination unit (10), a server provider service (35) and an equipment connector (37),
wherein the network-capable units are each designed to contact the server provider service (35) after they have been started up,
wherein the server provider service (35) is designed to transmit, to the network-capable units, contact information relating to the equipment connector (37) which manages the assignment of devices in the network (100),
wherein the network-capable units are also each designed to set up communication with the equipment connector (37) with the aid of the contact information transmitted by the server provider service (35) and to be individually identified by said equipment connector;
and wherein the equipment connector (37) is designed to transmit assignment information to the network-capable units.

11. Dental practice network according to Claim 10,
**characterized**
**in that** the network-capable units are designed to contact the server provider service (35) with the aid of a broadcast message.

12. Dental practice network according to Claim 10 or 11,
**characterized**
**in that** said network has a user interface, in particular a graphical user interface, which is preferably provided by the behind-the-head PC (20) and can be used to logically assign the network-capable units.

13. Dental practice network according to one of Claims 10 to 12,
**characterized**
**in that** said network has a memory for storing the assignment information, which memory is part of the equipment connector (37) or is connected to the latter.

14. Dental practice network according to one of Claims 10 to 13,
**characterized**
**in that** the server provider service (35) and the equipment connector (37) are implemented in a behind-the-head PC (20).

15. Dental practice network according to one of Claims 10 to 14,
**characterized**
**in that** the treatment or examination unit (10) has a display unit (17) for displaying operating options.

16. Dental practice network according to claim 15,
**characterized**
**in that** the display unit (17) is designed to display at least some of the operating options on the basis of HTML documents,
wherein the network (100) additionally has an HTTP server (39) for outputting the HTML documents.

17. Dental practice network according to one of Claims 10 to 16,
**characterized**
**in that** the behind-the-head PC (20) can be connected to at least one further examination or treatment device, for example an intraoral camera (40), wherein the connection is preferably effected via USB,
and wherein the behind-the-head PC (20) is designed to transmit information relating to a connected examination or treatment device to the equipment connector (37).

## Revendications

1. Procédé de configuration du réseau (100) d'un cabinet dentaire, dans lequel le réseau (100) présente au moins deux unités pouvant être mises en réseau, par exemple une unité d'examen ou de soins (10) dentaire ainsi qu'un PC dorsal (20) séparé de l'unité d'examen ou de soins (10), et dans lequel le procédé présente les étapes suivantes :
- le fait de contacter, par les unités pouvant être mises en réseau chacune après leur mise en service, un service de fournisseur de serveur (35) se trouvant dans le réseau,
- la transmission, par le service de fournisseur de serveur (35) aux unités pouvant être mises en réseau, d'informations de contact d'un connecteur d'équipements (37) gérant l'affectation d'appareils du réseau (100) et relié au réseau (100),
- l'établissement d'une communication entre les unités pouvant être mises en réseau et le connecteur d'équipements (37) et l'identification individuelle des unités pouvant être mises en réseau par le connecteur d'équipements (37), dans lequel l'établissement de la communication est effectué à l'aide des informations de contact transmises par le service de fournisseur de serveur (35) ;
- la détermination d'affectations des unités pouvant être mises en réseau et la transmission d'informations d'affectation par le connecteur d'équipements (37) aux unités pouvant être mises en réseau.

2. Procédé selon la revendication 1,
**caractérisé en ce**
**que** le fait de contacter le service de fournisseur de serveur (35) par les unités pouvant être mises en réseau est effectué respectivement à l'aide d'un message de diffusion.

3. Procédé selon la revendication 1 ou 2,
**caractérisé en ce**
**que** l'affectation des unités pouvant être mises en réseau est effectuée à l'aide d'une interface utilisateur, en particulier d'une interface utilisateur graphique, dans lequel l'interface utilisateur est mise à disposition de préférence par le PC dorsal (20).

4. Procédé selon l'une quelconque des revendications précédentes,
**caractérisé en ce**
**que** les informations d'affectation sont déposées dans une mémoire (38) qui est un élément constitutif du connecteur d'équipements (37) ou est reliée à celui-ci.

5. Procédé selon l'une quelconque des revendications précédentes,
**caractérisé en ce**
**que** le service de fournisseur de serveur (35) et le connecteur d'équipements (37) sont mis en oeuvre dans un PC dorsal (20).

6. Procédé selon l'une quelconque des revendications précédentes,
**caractérisé en ce**
**que** l'unité d'examen ou de soins (10) présente une unité d'affichage (17) pour la représentation d'options de commande.

7. Procédé selon la revendication 6,
**caractérisé en ce**
**que** l'unité d'affichage (17) est conçue pour représenter au moins une partie des options de commande sur la base de documents HTML, dans lequel le procédé comprend en plus l'étape suivante :
la transmission, à l'unité d'examen ou de soins (10) ou à l'unité d'affichage (17), d'informations de contact d'un serveur HTTP (39) qui est conçu pour la sortie des documents HTML.

8. Procédé selon la revendication 7,
**caractérisé en ce**
**que** la transmission des informations de contact du serveur HTTP (39) est effectuée par le service de fournisseur de serveur (35) ou le connecteur d'équipements (37).

9. Procédé selon l'une quelconque des revendications précédentes,
**caractérisé en ce**
**que** le PC dorsal (20) peut être relié à au moins un autre appareil d'examen ou de soins, par exemple une caméra intra-orale (40), dans lequel la liaison est effectuée de préférence par USB,
et dans lequel le PC dorsal (20) est conçu pour transmettre des informations, relatives à un appareil d'examen ou de soins branché, au connecteur d'équipements (37).

10. Réseau de cabinet dentaire (100) avec au moins deux unités pouvant être mises en réseau, par exemple une unité d'examen ou de soins (10) dentaire et un PC dorsal (20) séparé de l'unité d'examen ou de soins (10), un service de fournisseur de serveur (35) ainsi qu'un connecteur d'équipements (37),
dans lequel les unités pouvant être mises en réseau sont conçues chacune pour contacter, après leur mise en service, le service de fournisseur de serveur (35),
dans lequel le service de fournisseur de serveur (35) est conçu pour transmettre aux unités pouvant être mises en réseau des informations de contact du connecteur d'équipements (37) gérant l'affectation d'appareils du réseau (100),
dans lequel les unités pouvant être mises en réseau sont conçues en outre chacune pour établir, à l'aide des informations de contact transmises par le service de fournisseur de serveur (35), une communication avec le connecteur d'équipements (37) et pour être individuellement identifiées par celui-ci ;
et dans lequel le connecteur d'équipements (37) est conçu pour transmettre des informations d'affectation aux unités pouvant être mises en réseau.

11. Réseau de cabinet dentaire selon la revendication 10,
**caractérisé en ce**
**que** les unités pouvant être mises en réseau sont conçues pour contacter le service de fournisseur de serveur (35) à l'aide d'un message de diffusion.

12. Réseau de cabinet dentaire selon la revendication 10 ou 11,
**caractérisé en ce**
**que** celui-ci présente une interface utilisateur, en particulier une interface utilisateur graphique, qui est mise à disposition de préférence par le PC dorsal (20) et à l'aide de laquelle une affectation logique des unités pouvant être mises en réseau peut être réalisée.

13. Réseau de cabinet dentaire selon l'une quelconque des revendications 10 à 12,
**caractérisé en ce**
**que** celui-ci présente une mémoire pour le dépôt des informations d'affectation, qui est un élément constitutif du connecteur d'équipements (37) ou est reliée à celui-ci.

14. Réseau de cabinet dentaire selon l'une quelconque des revendications 10 à 13,
**caractérisé en ce**
**que** le service de fournisseur de serveur (35) et le connecteur d'équipements (37) sont mis en oeuvre dans un PC dorsal (20).

15. Réseau de cabinet dentaire selon l'une quelconque des revendications 10 à 14,
**caractérisé en ce**
**que** l'unité d'examen ou de soins (10) présente une unité d'affichage (17) pour la représentation d'options de commande.

16. Réseau de cabinet dentaire selon la revendication 15,
**caractérisé en ce**
**que** l'unité d'affichage (17) est conçue pour représenter au moins une partie des options de commande sur la base de documents HTML,
dans lequel le réseau (100) présente en plus un serveur HTTP (39) pour la sortie des documents HTML.

17. Réseau de cabinet dentaire selon l'une quelconque des revendications 10 à 16,
**caractérisé en ce**
**que** le PC dorsal (20) peut être relié à au moins un autre appareil d'examen ou de soins, par exemple une caméra intra-orale (40), dans lequel la liaison est effectuée de préférence par USB,
et dans lequel le PC dorsal (20) est conçu pour transmettre des informations, relatives à un appareil d'examen ou de soins branché, au connecteur d'équipements (37).
